# EUROPÄISCHE PATENTSCHRIFT

(11) **EP 1 660 054 B1**
(45) Veröffentlichungstag und Bekanntmachung des Hinweises auf die Patenterteilung: **13.08.2008**
(21) Anmeldenummer: 04735452.7
(22) Anmeldetag: 30.05.2004
(51) Int. Cl.: A61K 9/70

(54) **ERGOLINHALTIGES MITTEL ZUR TRANSDERMALEN APPLIKATION**
AGENT CONTAINING ERGOLIN FOR TRANSDERMAL APPLICATION
AGENT RENFERMANT DE L'ERGOLINE POUR APPLICATION TRANSDERMIQUE

(30) Priorität: 03.09.2003 DE 10341317
(43) Veröffentlichungstag der Anmeldung: 31.05.2006
(73) Patentinhaber: Axxonis Pharma AG, 10963 Berlin (DE)
(72) Erfinder: HOROWSKI, Reinhard, 13353 Berlin (DE); TACK, Johannes, 13595 Berlin (DE); BOSTEDT, Katalin Tisa, 82049 Pullach (DE); SCHENK, Dirk, 83623 Dietramszell (DE)
(74) Vertreter: Wablat, Wolfgang
(86) Internationale Anmeldenummer: PCT/DE2004/001133
(87) Internationale Veröffentlichungsnummer: WO 2005/025546

(56) Entgegenhaltungen:
- EP-A- 0 314 387
- WO-A-02/15890
- WO-A-02/078602
- DE-A- 10 054 713
- DE-A- 19 626 621

## Beschreibung

Die Erfindung betrifft ein Mittel zur transdermalen Applikation bestehend aus einer undurchlässigen Deckschicht, einer Ergolinverbindungen und gegebenenfalls penetrationsverstärkende Mittel enthaltenden Matrix, gegebenenfalls einer die Matrix abdeckenden Diffusionsbarriere, einer für diese Stoffe durchlässigen Kleberschicht und einer abziehbaren Schutzschicht. Die Ergolinderivate, bevorzugt Lisurid, in transdermalen therapeutischen Systemen sollen stabilisiert werden.

Bekannt sind Ergolinderivate enthaltende transdermale therapeutische Systeme zur Behandlung von Erkrankungen, die auf Störungen des dopaminergen Systems beruhen (WO 92/20339, WO 91/00746). Sie erscheinen als besonders geeignet zur Behandlung der Parkinsonschen Krankheit, des Parkinsonismus, des Restless-Legs-Syndroms, zur Prophylaxe des prämenstruellen Syndroms sowie als Mittel zur Laktationshemmung (DE 100 43 321). Mitunter werden sie auch zur Migräneprophylaxe vorgesehen, wo gleichfalls eine gut verträgliche, konstante Therapie erwünscht ist.

Transdermale therapeutische Systeme können beispielsweise als sogenannte Matrixsysteme aufgebaut sein. Die Matrixsysteme bestehen in der Regel aus einer undurchlässigen Deckschicht, einer Matrix, in welche die Wirkstoffformulierung eingebettet oder eingelöst ist, und - soweit die Matrix selbst nicht haftet - aus einer Haftkleberschicht sowie einer abziehbaren Schutzschicht.

Um einen definierten, gleichmäßigen Flux zu erreichen, wird der Wirkstoff üblicherweise mit geeigneten Hilfsstoffen, wie beispielsweise Lösungsmitteln, Penetrationsverstärkern und Kristallisationsinhibitoren kombiniert.

Es ist bekannt, daß transdermale therapeutische Systeme mit oxidationsempfindlichen Wirkstoffen wenig stabil sind. Eine Verbesserung der Stabilität dieser Systeme beschreibt beispielsweise DE 100 54 713 A1. Hierin werden die gesamten Formulierungsbestandteile des Systems so ausgewählt, daß die Summe ihrer Peroxidzahlen (als ein Maß für die Oxidierbarkeit) höchstens 20 beträgt. Dies bedeutet jedoch eine Einschränkung der in Frage kommenden Inhaltsstoffe oder eine aufwendige vorbereitende Behandlung der einzelnen Bestandteile mit Natriumhydrogensulfit-Lösungen zur Zerstörung der vorhandenen Peroxide.

Das Problem bei bisherigen Zubereitungen mit Ergolinderivaten ist jedoch die Instabilität des Wirkstoffs selbst. Transdermale therapeutische Systeme mit Ergolinderivaten zeigen nach einiger Zeit Verfärbungen in der Regel verbunden mit einem Abfall der Wirkstoffkonzentration. Ursache hierfür ist die recht hohe Oxidationsempfindlichkeit der Ergolinderivate. So wird beispielsweise Lisurid auch ohne Lichteinwirkung am Stickstoff in Position 6 des Ringsystems oxidiert.

Dies führt insbesondere bei der Langzeitanwendung zu Irritationen und Reizungen der Haut. Auch ist die kontrollierte Dosierung aufgrund der unbekannten Verringerung der Wirkstoffkonzentration nicht mehr möglich.

Die zur Stabilisierung üblicherweise eingesetzten Antioxidantien wie Citronensäure, Ascorbinsäure, Natriumsulfit, Natriumdisulfit, Alkylgallate, Ascorbylpalmitat u.ä. ergeben keine wesentliche Verbesserung.

Aufgabe der vorliegenden Erfindung ist die Schaffung eines ein Ergolinderivat enthaltenden, lagerstabilen, transdermalen therapeutischen Systems, in dem der oxidative Abbau des Wirkstoffes verhindert wird und das damit ohne Reizungen oder Irritationen auch über längere Zeiträume auf der Haut verbleiben kann.

Erfindungsgemäß wird diese Aufgabe durch ein Mittel gemäß Anspruch 1 gelöst. Ergolinderivate werden in einem transdermalen therapeutischen System durch die Kombination aus mindestens einem fettlöslichen, radikalfangenden Antioxidans ausgewählt aus Di-tert.-butylmethylphenolen, Di-*tert*.-butylmetoxyphenolen, Tocopherolen oder Ubichinonen und einem basischen Polymer stabilisiert.

Untersuchungen haben gezeigt, daß die Gegenwart eines der oben genannten Antioxidantien allein noch keine signifikante Verbesserung der Stabilität der Ergolinderivate bewirkt.

Transdermale therapeutische Systeme, in denen neben den oben genannten Antioxidantien auch als basisches Polymer Butylmethacrylat-(2-Dimethylaminoethyl)metharcylat-Methylmethacrylat-Copolymer (Eudragit E 100 der Firma Röhm, Deutschland) vorliegt, zeigen eine überraschend hohe Stabilität. Hierbei kann das basisches Polymer auch in einem Gemisch mit anderen üblichen Polymeren wie z.B neutralen Polyacrylaten vorliegen. Darüberhinaus kann das Polymergemisch zur Verbesserung der Klebkraft auch übliche Klebkraftverstärker (beispielsweise Harze oder Polyacrylate) enthalten.

Die erfindungsgemäßen Systeme haben üblicherweise ein Flächengewicht von 2 bis 10 mg/cm². Dieses ergibt sich aus der Summe aller Bestandteile nach dem Trocknen. Der Gehalt an matrixbildenden Polymeren beläuft sich insgesamt auf 50 bis 95 Gew.-%, vorzugsweise 60 bis 85 %. Der Anteil anderer Polymere beträgt 5 bis 30 Gew.-%, vorzugsweise 10 bis 20 %. Der Gehalt an Antioxidans liegt zwischen 0,25 und 5 Gew.-%, bevorzugt 0,5 bis 1,5 %. Der Wirkstoffanteil liegt bei 1 bis 10 Gew.-%, bevorzugt bei 3 bis 6 %.

Die erfindungsgemäßen Kombinationen haben einen unerwarteten synergistischen Effekt, der die Oxidation der Ergolinderivate in transdermalen Systemen behindert.

### Ausführungsbeispiele:

### Beispiel 1

Es wurden Stabilitätsuntersuchungen mit Proben, die Kombinationen aus unterschiedlichen Antioxidantien und Polymeren enthielten, gemacht.

Hierbei wurde Lisurid als Wirkstoff eingesetzt. Darüber hinaus enthalten die Proben weitere Hilfsstoffe, die in transdermalen therapeutischen Systemen üblicherweise verwendet werden.

### Probenherstellung:

In 900 g einer ca. 50%igen Lösung von Polymer-Klebstoff in einer Mischung aus 2-Propanol und Aceton werden nacheinander 150 g Polyvidon und 300 g Dibutylsebacat als Weichmacher und 20 g Floral E 105 (hydrierte Kolophoniumpentaerthritester der Firma Hercules) als Klebkraftverstärker unter Rühren bei Raumtemperatur eingebracht. Danach werden 50 g Lisurid und 15 g Antioxidans in einem Teil des Lösungsmittels vorsuspendiert und unter fortlaufendem Rühren der Klebstofflösung zugefügt. Nach dem vollständigen Auflösen wird die Lösung mit Aceton auf das Endgewicht gebracht und zur Entfernung der Gasblasen ca. 24 Stunden stehen gelassen. Anschließend wird die Lösung mit Hilfe einer geeigneten Beschichtungsvorrichtung (z.B. Knife over Roll) auf eine silikonisierte Polyesterfolie (Liner-Folie) aufgetragen, so daß nach dem Entfernen der flüchtigen Lösungsmittel bei 40 bis 90°C ein gleichmässiger Film mit einem Flächengewicht von ca. 5 mg/cm² entsteht. Danach wird mit einer Abdeckfolie aus Polyester kaschiert. Das so erhaltene Laminat wird mittels einer geeigneten Stanzvorrichtung in Einzelpflaster mit einer Fläche von 10 cm² geteilt und in lichtundurchlässige Beutel aus einem Aluminium/Papier-Verbundfolie eingebracht.

Tabelle 1 zeigt die Zusammensetzung der untersuchten Proben.

**Tabelle 1: Zusammensetzungen der Proben in Gew.-%**

| Probennummer | #80 | #81 | #82 | #83 | #84 | #85 | #86 | #87 | #88 | #90 | #98 |
|---|---|---|---|---|---|---|---|---|---|---|---|
| Lisurid | 3,3 | 3,2 | 4,0 | 4,0 | 4,0 | 4,0 | 4,0 | 4,0 | 4,0 | 3,0 | 3,0 |
| MA24A¹⁾ | 44,9 | 45,1 | - | - | - | - | - | - | - | 53,0 | 52,0 |
| Eudragit E 100²⁾ | - | - | - | - | 85,0 | 85,0 | 68,0 | 68,0 | 60,0 | - | - |
| Durotac DT 387-2510³⁾ | - | - | 77,0 | 77,0 | - | - | 17,0 | 17,0 | 15,0 | - | - |
| Ascorbylpalmitat | - | - | - | - | - | - | - | - | - | - | 2,0 |
| Tocopherol | 1,0 | - | 1,0 | - | 1,0 | - | 1,0 | - | 1,0 | 1,0 | - |
| BHT⁴⁾ | - | 0,9 | - | 1,0 | - | 1,0 | - | 1,0 | - | - | - |
| Polyvidon | 9,2 | 9,3 | 10,0 | 10,0 | 10,0 | 10,0 | 10,0 | 10,0 | 20,0 | 10,0 | 10,0 |
| Transcutol⁵⁾ | 27,0 | 27,0 | - | - | - | - | - | - | - | 25,0 | 25,0 |
| Eutanol⁶⁾ | 8,7 | 8,6 | 5,0 | 5,0 | - | - | - | - | - | 8,0 | 8,0 |
| Dimethylacetamid | 5,9 | 5,9 | - | - | - | - | - | - | - | - | - |

| | | | | | | | | | | | |
|---|---|---|---|---|---|---|---|---|---|---|---|
| ¹⁾ Polyisobutylen der Firma Adhesive Research, Irland ²⁾ Butylmethacrylat-2-(Dimethylamino)ethylmethacrylat-Methylmethacrylat-Copolymer (1:2:1) der Firma Röhm, Deutschland ³⁾ neutrales Polyacrylat der Firma National Starch, USA ⁴⁾ Butylhydroxytoluol (2,6-Di-*tert.*-butyl-4-methylphenol) ⁵⁾ Diethylenglykolmonoethylether der Firma Gattefossé, Frankreich ⁶⁾ 2-Hexyldecanol der Firma Cognis, Deutschland | | | | | | | | | | | |

### Lagerung:

Die Proben wurden unter den folgenden Bedingungen gelagert:
a) bei 4 ° C,
b) bei 25°C und 60% Luftfeuchte,
c) bei 40°C und 75% Luftfeuchte.

Nach einem Monat Lagerzeit wurde die Konzentration des Aminoxids, das durch Oxidation am Stickstoff in Position 6 des ergolinen Ringsystems erhalten wurde (Lisurid-N-oxid), bestimmt.

### Bestimmung des Aminoxidgehalts:

Die Menge des Aminoxids wurde mittels einer HPLC-Methode bestimmt, die folgende Parameter aufweist:

| | |
|---|---|
| Säule: | Luna C18(II), 100 mm x 4,6 mm ID |
| Vorsäule: | Phenomenex C18, 4 mm x 3 mm ID |
| Säulentemperatur: | 35°C |
| Laufzeit: | 30 min |
| Flussrate: | 1,20 ml/min |
| mobile Phase: | A: 10 mM TRIS-Puffer, pH 8,7 |
| | B: Acetonitril |
| Gradientenprofil: | 0 bis 25. Minute: 12% B |
| | 25. bis 27. Minute: 42% B |
| | 28. bis 38. Minute: 12% B |
| Detektion: | Fluoreszenz-Detektor |

### Probenvorbereitung:

Ein Lisuridplaster, das wie in Beispiel 1 beschrieben hergestellt wurde, wird nach Wiegen und Entfernen der Liner-Folie in 50 ml Lösungsmittel (2-Propanol) 15 Minuten lang geschüttelt. Anschließend werden 5 ml der Lösung mit Diluent (Acetonitril) auf 20 ml verdünnt. Etwa 2 ml dieser Lösung werden 2 Minuten bei 5000 UPM zentrifugiert und die klare, überstehende Lösung in ein HPLC-Probenvial abgefüllt.

Tabelle 2 zeigt die Ergebnisse.

**Tabelle 2: Die Bildung von Aminoxid aus Lisurid nach einmonatiger Lagerzeit**

| Probe | Gehalt an Lisurid-N-oxid in Gew.-% | | |
|---|---|---|---|
| | a: 4°C | b: 25°C | c: 40°C |
| #80: MA24A/Tocopherol | 0,51 | 1,59 | 2,80 |
| #81: MA24A/BHT | 0,45 | 1,26 | 1,86 |
| #82: Durotac/Tocopherol | 0,70 | 1,21 | 1,49 |
| #83: Durotac/BHT | 0,71 | 1,08 | 1,44 |
| #84: Eudragit/Tocopherol | 0 | 0,11 | 0,26 |
| #85: Eudragit/BHT | 0,06 | 0,09 | 0,22 |
| #86: Eudragit/Durotac/Tocopherol | 0 | 0,14 | 0,37 |
| #87:Eudragit/Durotac/BHT | 0 | 0,14 | -- |
| #88:Eudragit/Durotac/Tocopherol | 0,11 | 0,21 | 0,44 |
| #90:MA24A/Tocopherol | -- | -- | 1,93 |
| #98:MA24A/Ascorbylpalmitat | 0,27 | 0,58 | -- |

### Beispiel 2

Es wurden Stabilitätsuntersuchungen mit Proben, die Kombinationen aus unterschiedlichen Antioxidantien mit basischen Polyacrylaten enthielten, gemacht.

Als Wirkstoff wurde Lisurid eingesetzt. Darüber hinaus enthalten die Proben weitere Hilfsstoffe, die in transdermalen therapeutischen Systemen üblicherweise verwendet werden.

### Probenherstellung:

In 1800 g einer ca. 45%igen Lösung von basischem Polyacrylat-Klebstoff in Aceton werden nacheinander 175 g Polyvidon und 310 g Dibutylsebacat als Weichmacher sowie 175 g Dodecanol als Cosolvens unter Rühren bei Raumtemperatur eingebracht. Danach werden 80 g Lisurid und 17 g Antioxidans in einem Teil der Lösungsmittels vorsuspendiert und der Klebstofflösung hinzugefügt. Nach dem vollständigen Auflösen werden 35 g Floral als Tackifier hinzugefügt. Die Lösung wird mit Aceton auf das Endgewicht gebracht und zur Entfernung der Gasblasen ca. 24 Stunden stehen gelassen. Anschließend wird die Lösung mit Hilfe einer geeigneten Beschichtungsvorrichtung (z.B. Knife over Roll) auf eine silikonisierte Polyesterfolie (Liner-Folie) aufgetragen, so daß nach dem Entfernen der flüchtigen Lösungsmittel bei 40 bis 90°C ein gleichmässiger Film mit einem Flächengewicht von etwa 5 mg/cm² entsteht. Danach wird mit einer Abdeckfolie aus Polyester kaschiert. Das so erhaltene Laminat wird mittels einer geeigneten Stanzvorrichtung in Einzelpflaster mit einer Fläche von 10 cm² geteilt und in lichtundurchlässige Beutel aus Aluminium/Papier-Verbundfolie eingebracht.

Tabelle 3 zeigt die Zusammensetzung der Proben.

**Tabelle 3: Zusammensetzung der Proben in Gew.-%**

| Probennummer | #C005 | #151 | #156 | #C001 | #152 |
|---|---|---|---|---|---|
| Lisurid | 5,0 | 5,0 | 5,0 | 4,0 | 5,0 |
| Polyvidon | 10,0 | 10,0 | 10,0 | 10,0 | 10,0 |
| Laurylalkohol | 15,0 | 15,0 | 15,0 | - | 15,0 |
| Foral 105 E¹⁾ | 2,0 | 2,0 | 2,0 | - | 2,0 |
| BHT²⁾ | - | - | 1,0 | 0,4 | - |
| Natriumsulfit | - | - | - | - | 0,1 |
| Eudragit E 100³⁾ | 68,0 | 68,0 | 67,0 | 85,6 | 67,9 |

| | | | | | |
|---|---|---|---|---|---|
| ¹⁾ hydrierte Kolophoniumpentaerythritester der Firma Hercules ²⁾ Butylhydroxytoluol ³⁾ Butylmethacrylat-2-(Dimethylamino)ethylmethacrylat-Methylmethacrylat-Copolymer (1:2:1) der Firma Röhm, Deutschland | | | | | |

### Lagerung:

Die Proben wurden bei 25°C und 60% Luftfeuchte und bei 40°C und 75% Luftfeuchte gelagert. Es wurde nach 1 und nach 3 Monaten Lagerzeit die Konzentration des Aminoxids bestimmt.

### Bestimmung des Aminoxidgehalts:

Der Aminoxidgehalt der Proben wurde mit der in Beispiel 1 beschriebenen HPLC-Methode bestimmt.

Tabelle 4 zeigt die Ergebnisse der Stabilitätstests.

**Tabelle 4: Die Bildung von Aminoxid aus Lisurid nach ein-und dreimonatiger Lagerzeit**

| Probe | Gehalt an Lisurid-N-oxid in Gew.-% | | | |
|---|---|---|---|---|
| | 25°C 1 Monat | 25°C 3 Monate | 40°C 1 Monat | 40°C 3 Monate |
| #C005: Eudragit | 0,20 | 0,50 | 1,18 | 3,51 |
| #151: Eudragit | 0,21 | 0,40 | 0,91 | 2,43 |
| #153: Eudragit | 0,21 | 0,48 | 0,92 | 2,00 |
| #156: Eudragit/BHT | 0,14 | - | 0,27 | - |
| #C001: Eudragit/BHT | 0,10 | 0,14 | 0,38 | 0,44 |
| #152: Eudragit/Natriumsulfit | 0,18 | 0,36 | 0,82 | 2,23 |

## Patentansprüche

1. Mittel zur transdermalen Applikation bestehend aus einer undurchlässigen Deckschicht, einer Ergolinverbindungen und gegebenenfalls penetrationsverstärkende Mittel enthaltenden Matrix, gegebenenfalls einer die Matrix abdeckenden Diffusionsbarriere, einer für diese Stoffe durchlässigen Kleberschicht und einer abziehbaren Schutzschicht, **dadurch gekennzeichnet, dass** die Ergolinverbindungen durch ein Antioxidans, ausgewählt aus der Gruppe von Di-*tert.*-butylmethylphenolen, tert.-Butylmethoxyphenolen, Tocopherolen und/oder Ubichinonen und ein Butylmethacrylat-2-(Dimethylamino)ethylmethacrylat-Methylmethacrylat-Copolymer stabilisiert sind.

2. Mittel nach Anspruch 1, **dadurch gekennzeichnet, dass** das Antioxidans eine Verbindung ist, die mit freien Radikalen reagiert.

3. Mittel nach einem der Ansprüche 1 bis 2, **dadurch gekennzeichnet, dass** das Antioxidans in Mengen von 0,25 Gew.-% bis 5 Gew.-% vorliegt.

4. Mittel nach einem der Ansprüche 1 bis 3, **dadurch gekennzeichnet, dass** das Butylmethacrylat-2-(Dimethylamino)ethylmethacrylat-Methylmethacrylat-Copolymer in der Matrix oder der Kleberschicht enthalten ist.

5. Mittel nach einem der Ansprüche 1 bis 4, **dadurch gekennzeichnet, dass** das Butylmethacrylat-2-(Dimethylamino)ethylmethacrylat-Methylmethacrylat-Copolymer in der Matrix oder der Kleberschicht einen Klebkraftverstärker enthält.

6. Mittel nach einem der Ansprüche 1 bis 5, **dadurch gekennzeichnet, dass** der Klebkraftverstärker Harze und/oder neutrale Polyacrylate enthält.

7. Mittel nach einem der Ansprüche 1 bis 6, **dadurch gekennzeichnet, dass** es 1 bis 20 Gew.-% Klebkraftverstärker enthält.

8. Mittel nach einem der Ansprüche 1 bis 7, **dadurch gekennzeichnet, dass** es 2 bis 10 Gew.-% Klebkraftverstärker enthält.

9. Mittel nach einem der Ansprüche 1 bis 8, **dadurch gekennzeichnet, dass** die Ergolinverbindung Lisurid oder Protergurid ist.

10. Verwendung eines Mittels nach einem der Ansprüche 1 bis 9 zur Herstellung eines Arzneimittels zur Prophylaxe und Therapie von dopaminerg behandelbaren Krankheiten.

11. Verwendung eines Mittels nach einem der Ansprüche 1 bis 10 zur Herstellung eines Arzneimittels zur Behandlung der Parkinsonschen Krankheit, zur Behandlung und Prävention des Restless-Legs-Syndroms und des prämenstruellen Syndroms sowie zur Laktationshemmung und zur Migräneprophylaxe.

## Claims

1. An agent for transdermal application consisting of an impermeable top layer, a matrix containing ergoline compounds and, optionally, penetration-enhancing agents, optionally a diffusion barrier covering the matrix, an adhesive layer that is permeable to these compounds, and a peelable protective layer, **characterized in that** the ergoline compounds are stabilized by an antioxidant, which is selected from the group of di-tert.-butyl methyl phenols, tert.-butyl methoxyphenols, tocopherols, and/or ubiquinones and a butyl methacrylate-2-(dimethylamino)ethylmethacrylate-methacrylate copolymer.

2. The agent according to claim 1 **characterized in that** the antioxidant is a compound that reacts with free radicals.

3. The agent according to any one of claims 1 to 2 **characterized in that** the antioxidant is present in quantities from 0.25% by weight to 5% by weight.

4. The agent according to any one of claims 1 to 3 **characterized in that** the butyl methacrylate-2-(dimethylamino)ethyl-methacrylate-methacrylate copolymer is contained in the matrix or in the adhesive layer.

5. The agent according to any one of claims 1 to 4 **characterized in that** the butyl methacrylate-2-(dimethylamino)ethyl-methacrylate-methacrylate copolymer in the matrix or in the adhesive layer contains a tackifier.

6. The agent according to any one of claims 1 to 5 **characterized in that** the tackifier contains resins and/or neutral polyacrylates.

7. The agent according to any one of claims 1 to 6, **characterized in that** it contains 1 to 20% by weight tackifier.

8. The agent according to any one of claims 1 to 7, **characterized in that** it contains 2 to 10% by weight tackifier.

9. The agent according to any one of claims 1 to 8 **characterized in that** the ergoline compound is lisuride or proterguride.

10. Use of an agent according to any one of claims 1 to 9 for the preparation of a medicinal product for prophylaxis and treatment of dopaminergically treatable diseases.

11. Use of an agent according to any one of claims 1 to 10 for the preparation of a medicinal product for the treatment of Parkinson's disease, for the treatment and prevention of restless legs syndrome and the premenstrual syndrome, as well as for lactation inhibition and migraine prophylaxis.

## Revendications

1. Produit destiné à l'application transdermique composé d'une couche superficielle imperméable, d'une matrice comprenant des combinaisons d'ergoline et le cas échéant de produits renforçant la pénétration, le cas échéant d'une barrière de diffusion recouvrant la matrice, d'une couche d'adhésif perméable à ces matières et d'une couche protectrice pelable, **caractérisé en ce que** les combinaisons d'ergoline sont stabilisées par un antioxydant, choisi dans le groupe des di-tert-butylméthylphénols, tert.-butylméthoxyphénols, tocophérols et/ou ubiquinones et par un copolymère de butylméthacrylate-2-(diméthylamino)éthylméthacrylate-méthylmethacrylate.

2. Produit selon la revendication 1, **caractérisé en ce que** l'antioxydant est une combinaison réagissant aux radicaux libres.

3. Produit selon l'une quelconque des revendications 1 à 2, **caractérisé en ce que** l'antioxydant est présent dans des quantités de 0,25 % en poids à 5 % en poids.

4. Produit selon l'une quelconque des revendications 1 à 3, **caractérisé en ce que** le copolymère de butylméthacrylate-2-(diméthylamino)éthylméthacrylate-méthylmethacrylate est contenu dans la matrice ou dans la couche adhésive.

5. Produit selon l'une quelconque des revendications 1 à 4, **caractérisé en ce que** le copolymère de butylméthacrylate-2-(diméthylamino)éthylméthacrylate-méthylmethacrylate contient dans la matrice ou dans la couche adhésive un renforçateur de pouvoir adhésif.

6. Produit selon l'une quelconque des revendications 1 à 5, **caractérisé en ce que** le renforçateur de pouvoir adhésif contient des résines ou des polyacrylates neutres.

7. Produit selon l'une quelconque des revendications 1 à 6, **caractérisé en ce qu'**il contient de 1 à 20 % en poids de renforçateur de pouvoir adhésif.

8. Produit selon l'une quelconque des revendications 1 à 7, **caractérisé en ce qu'**il contient de 2 à 10 % en poids de renforçateur de pouvoir adhésif.

9. Produit selon l'une quelconque des revendications 1 à 8, **caractérisé en ce que** la combinaison d'ergoline est du Lisuride ou du Proterguride.

10. Utilisation d'un produit selon l'une quelconque des revendications 1 à 9 pour la fabrication d'un produit pharmaceutique pour la prophylaxie et la thérapie de maladies susceptibles d'être traitées par des agonistes dopaminergiques.

11. Utilisation d'un produit selon l'une quelconque des revendications 1 à 10 pour la fabrication d'un produit pharmaceutique pour le traitement de la maladie de Parkinson, pour le traitement et la prévention du syndrome d'impatience musculaire et du syndrome prémenstruel, ainsi que pour inhiber la lactation et pour la prophylaxie de la migraine.
